# EUROPEAN PATENT APPLICATION

(11) **EP 4 737 977 A1**
(43) Date of publication of application: **06.05.2026**
(21) Application number: 24825950.9
(22) Date of filing: 19.06.2024
(51) Int. Cl.: G02B 21/32, B25J 7/00, C12M 1/00, G02B 21/36

(54) **MANIPULATION SYSTEM AND MANIPULATION METHOD**

(30) Priority: 20.06.2023 JP 2023100574
(71) Applicant: NATIONAL UNIVERSITY CORPORATION TOKAI NATIONAL HIGHER EDUCATION AND RESEARCH SYSTEM, Nagoya-shi, Aichi 464-8601 (JP); Inter-University Research Institute Corporation Research Organization of Information and Systems, Tachikawa-shi, Tokyo 190-0014 (JP)
(72) Inventor: AOYAMA, Tadayoshi, Nagoya-shi Aichi 464-8601 (JP); SAKAMOTO, Kazuya, Nagoya-shi Aichi 464-8601 (JP); MORI, Ryoya, Nagoya-shi Aichi 464-8601 (JP); KOBAYASHI, Taisuke, Tokyo 101-8430 (JP)
(74) Representative: Seemann & Partner Patentanwälte mbB
(86) International application number: PCT/JP2024/022272
(87) International publication number: WO 2024/262549

(57) **Abstract**

A manipulation system (310) includes a control apparatus (330). The control apparatus (330) includes: a movement amount acquisition unit (56) that acquires a movement amount of the manipulator; a position identification unit (58) that identifies a position of the sample and a position of the manipulator based on an image captured by the imaging apparatus (24); and a calculation unit (376) that calculates a target position or target movement amount of the manipulator for performing a predetermined operation on the sample based on the position of the sample and the position or movement amount of the manipulator.

## Description

### TECHNICAL FIELD

The present disclosure relates to manipulation systems and manipulation methods.

### BACKGROUND ART

Manipulation systems for manipulating samples such as cells by using a manipulator are known. Since cells are miniscule, cells are manipulated while the positions of the cells and the manipulator are observed under a microscope. For example, a technology has been proposed that is for improving the operability of a sample by generating force information indicating a magnitude of a force sensation presented to a user based on an image captured of the sample and presenting the force sensation to the user via a force sensation presentation apparatus.

### RELATED-ART LITERATURE

### PATENT LITERATURE

Patent Literature 1: WO 2023/027095

### SUMMARY OF INVENTION

### TECHNICAL PROBLEM

In intracytoplasmic sperm injection, in which a sperm is injected into an egg cell using a pipette, manipulation to rotate the cell in three dimensions is required as a preliminary preparation for inserting the pipette into the cells while avoiding the vicinity of the polar body of the cell. Skilled cell manipulation operators can skillfully rotate cells while minimizing cell damage based on two-dimensional visual information from a microscope. However, for beginners in cell manipulation, it is not easy to rotate and manipulate cells properly based only on visual information, and it usually takes a long time (for example, one year or more) to master the skill.

The present disclosure addresses the issue described above, and an illustrative purpose is to provide a technology for improving the operation accuracy when performing a predetermined operation on a sample using a manipulator.

### SOLUTION TO PROBLEM

A manipulation system according to one embodiment of the present disclosure includes: a manipulator for manipulating a sample; a manipulator drive mechanism for moving the manipulator; an imaging apparatus that images the sample and the manipulator through an objective lens; and a control apparatus. The control apparatus includes: a movement amount acquisition unit that acquires a movement amount of the manipulator; a position identification unit that identifies a position of the sample and a position of the manipulator based on an image captured by the imaging apparatus; and a calculation unit that calculates a target position or target movement amount of the manipulator for performing a predetermined operation on the sample based on the position of the sample and the position or movement amount of the manipulator.

Another embodiment of the present disclosure relates to a manipulation method. This manipulation method includes: acquiring an image of a sample and a manipulator captured through an objective lens; acquiring a movement amount of the manipulator; identifying a position of the sample and a position of the manipulator based on the image acquired; and calculating a target position or target movement amount of the manipulator for performing a predetermined operation on the sample based on the position of the sample and the position or movement amount of the manipulator.

Optional combinations of the aforementioned constituting elements, and constituting elements and implementations of the disclosure in the form of methods, systems, programs, etc., may also be practiced as additional modes of the present disclosure.

### ADVANTAGEOUS EFFECTS OF INVENTION

According to the present disclosure, the operation accuracy when performing a predetermined operation on a sample using a manipulator can be improved.

### BRIEF DESCRIPTION OF DRAWINGS

Fig. 1 is a diagram schematically showing a configuration of the manipulation system according to the first embodiment.
Fig. 2 is a block diagram schematically showing a functional configuration of an image recognition device according to the first embodiment.
Figs. 3(a) to 3(c) are diagrams schematically showing how a sample is manipulated by a manipulator.
Fig. 4(a) is a diagram showing an example of an operation trajectory of the manipulator when a skilled operator performs a predetermined operation, and Fig. 4(b) is a diagram showing an example of a reference trajectory set around the sample.
Figs. 5(a) and 5(b) are diagrams schematically showing a calculation method for the second movement amount.
Figs. 6(a) and 6(b) are diagrams schematically showing an example of setting a weight coefficient for calculating a target movement amount.
Fig. 7 is a diagram showing an example of operation support information displayed on a display apparatus.
Fig. 8 is a diagram showing an example of the operation support information displayed on the display apparatus.
Figs. 9(a) to 9(c) are diagrams showing an example of operation trajectories of the manipulator when a beginner performs a predetermined operation.
Fig. 10 is a flowchart showing an example of a manipulation method according to the first embodiment.
Fig. 11 is a block diagram schematically showing a functional configuration of a control apparatus according to the second embodiment.
Fig. 12 is a diagram showing an example of the operation support information displayed on the display apparatus.
Fig. 13 is a diagram showing an example of the operation support information displayed on the display apparatus.
Fig. 14 is a flowchart showing an example of a manipulation method according to the second embodiment.
Fig. 15 is a block diagram schematically showing a functional configuration of a control apparatus according to the third embodiment.
Fig. 16 is a flowchart showing an example of a manipulation method according to the third embodiment.
Fig. 17 is a block diagram schematically showing a functional configuration of a control apparatus according to the fourth embodiment.
Fig. 18 is a flowchart showing an example of a manipulation method according to the fourth embodiment.

### DESCRIPTION OF EMBODIMENTS

First, an overview of the present disclosure will be described. The present disclosure relates to a manipulation system. The manipulation system includes an optical microscope for observing a sample such as a cell and a manipulator for manipulating the sample. The user manipulates the sample by moving the manipulator while observing the sample and the manipulator with an optical microscope. The manipulation system is used, for example, for intracytoplasmic sperm injection (ICSI) where a sperm is injected into an egg cell. In ICSI, the manipulation involves i) an operation of holding an egg cell, ii) an operation of rotating the egg cell in a predetermined orientation, and iii) an operation of inserting a pipette into the egg cell to inject a sperm. Of these operations, the rotating operation in ii) is more difficult and requires a skillful technique to properly rotate the cell while minimizing cell damage.

The present disclosure provides a technology for supporting a predetermined operation through manipulation, such as a rotational operation of cells and improving the operation accuracy when performing the predetermined operation. In the present disclosure, the next position (also referred to as target position) or movement amount (also referred to as target movement amount) of a manipulator for a predetermined operation is calculated using the position of a sample and the position or movement amount of the manipulator as input. By superimposing and displaying the target position or the target movement amount on a captured image of the sample and manipulator, it is possible to assist the operation of moving the manipulator to a desired position. Further, by presenting a force sensation in the direction of moving the manipulator toward the target position via a force sensation presentation apparatus, it is possible to assist the operation of moving the manipulator to a preferred position. For example, the present disclosure can help beginners with little experience in cell manipulation to acquire the skill.

### (First embodiment)

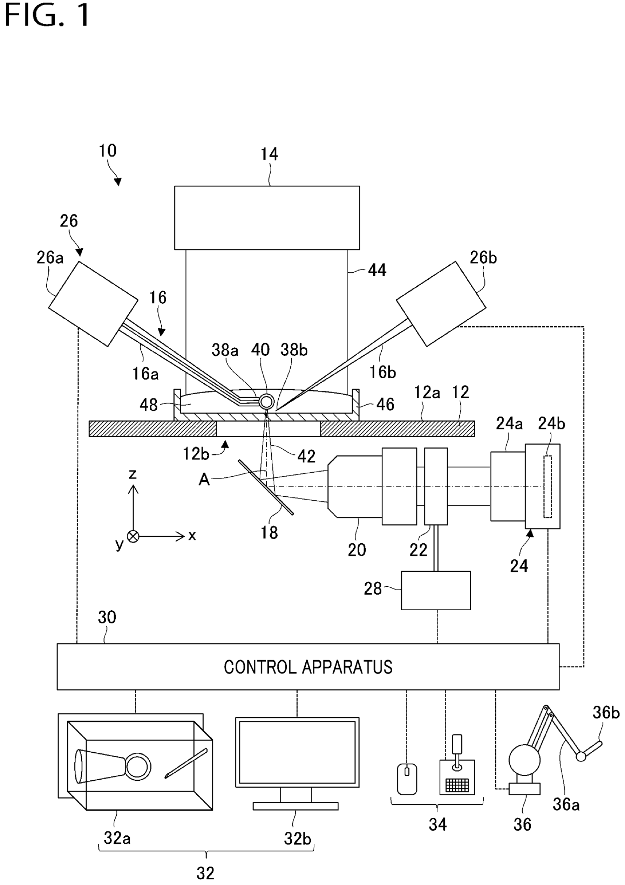

Fig. 1 schematically shows a configuration of a manipulation system 10 according to the first embodiment. The manipulation system 10 includes an inverted microscope configuration. The manipulation system 10 includes a stage 12, an illumination apparatus 14, a manipulator 16, a redirecting mirror 18, an objective lens 20, a variable focus lens 22, an imaging apparatus 24, a manipulator drive mechanism 26, a lens drive mechanism 28, a control apparatus 30, a display apparatus 32, an input apparatus 34, and a force sensation presentation apparatus 36.

Referring to Fig. 1, a coordinate system is set with reference to an optical axis A of the objective lens 20 on the stage 12. The direction in which the optical axis A of the objective lens 20 extends on the stage 12 is the z direction, and the directions perpendicular to the optical axis A are the x direction and the y direction. In the example shown in Fig. 1, the direction in which the optical axis A of the objective lens 20 extends on the stage 12 coincides with the direction perpendicular to a support surface 12a of the stage 12. It should be noted that the direction in which the optical axis A of the objective lens 20 extends on the stage 12 may be deviated from the direction perpendicular to the support surface 12a of the stage 12.

The stage 12 has a support surface 12a for horizontally supporting the sample 40 and an opening 12b for transmitting an observation light 42 from the sample 40. There is no particular restriction on the sample 40 to be manipulated; however, cells such as those of humans and animals can be the subject of manipulation. The sample 40 is, for example, contained in a sample dish 46 made of a transparent material such as resin and glass, and the sample dish 46 is arranged on stage 12. The sample 40 is, for example, suspended in a liquid 48 such as water contained in the sample dish 46.

The illumination apparatus 14 is provided above the stage 12 and illuminates the sample 40 on the stage 12. The illumination apparatus 14 projects an illumination light 44 such as white light toward the sample 40. The illumination apparatus 14 is configured to provide transillumination. The illumination apparatus 14 may be capable of projecting a visible illumination light 44 of a specific wavelength selected for fluorescent observation, etc. The illumination apparatus 14 projects, for example, the illumination light 44 that provides a uniform illuminance distribution on the stage 12.

The manipulator 16 is provided on the stage 12 and is used to manipulate the sample 40. In the example shown in Fig. 1, the manipulator 16 includes a holding pipette 16a and an injection pipette 16b. For example, the holding pipette 16a is used to fix a cell, and the injection pipette 16b is used for cell manipulation such as gene transfer into the cell. In the example shown in Fig. 1, two manipulators are provided; however, the number of manipulators may be one or may be three or more.

The redirecting mirror 18 is provided directly below the opening 12b of the stage 12. The redirecting mirror 18 is arranged so as to reflect the observation light 42 from the sample 40 toward the objective lens 20. In the illustrated example, the optical axis A of the objective lens 20 is folded back by the redirecting mirror 18; however, the redirecting mirror 18 may not be provided, and the objective lens 20 may be arranged on an optical axis extending in the z direction.

The objective lens 20 is arranged at a position where the observation light 42 from the redirecting mirror 18 is incident. The objective lens 20 is arranged at a position distanced from the redirecting mirror 18 in the +x direction. The objective lens 20 preferably has a relatively long working distance (WD). The specification such as magnification factor and working distance of the objective lens 20 is not particularly limited. For example, an ultra-long working objective lens having a working distance of 20 mm-40 mm at a magnification factor of 10 times-50 times can be used.

The variable focus lens 22 is arranged at a position where the observation light 42 transmitted through the objective lens 20 is incident. The variable focus lens 22 is arranged between the objective lens 20 and the imaging apparatus 24 and is arranged, for example, adjacent to or in close proximity to the objective lens 20. The variable focus lens 22 is configured to have refractive power that is variable within a predetermined range. The variable focus lens 22 may be a convex lens having only positive refractive power, a concave lens having only negative refractive power, or may be configured to have positive or negative refractive power switchably.

The variable focus lens 22 is composed of, for example, a liquid lens, and is configured so that the focal length is variable by deforming a flexible transparent membrane that seals the liquid lens. The shape of the transparent membrane is controlled by varying the pressure applied to the transparent membrane. For example, the focal length of the variable focus lens 22 can be electrically controlled by using an electromagnetic actuator or a piezoelectric device. The variable focus lens 22 is configured, for example, so that the effective working distance in the combination of the objective lens 20 and the variable focus lens 22 is variable in a range of about 2 mm.

The imaging apparatus 24 generates a captured image by imaging the observation light 42 transmitted through the variable focus lens 22. The imaging apparatus 24 includes an imaging lens 24a and an imaging device 24b. The imaging lens 24a forms an image of the observation light 42 on the imaging device 24b. The imaging device 24b is an image sensor such as a CMOS sensor and is capable of generating a captured image at a high frame rate. The frame rate of the imaging apparatus 24 is not particularly limited but is preferably 100 frames per second or more and, more preferably, 500 frames per second or more.

The objective lens 20, the variable focus lens 22, and the imaging apparatus 24 are arranged along the optical axis A extending in the x direction and are fixed to, for example, a lens barrel extending in the x direction. An additional redirecting mirror not shown may be provided between the variable focus lens 22 and the imaging apparatus 24 to further fold back the optical axis A.

The manipulator drive mechanism 26 moves the manipulator 16 to make the three-dimensional position of the manipulator 16 variable. In the example shown in Fig. 1, the manipulator drive mechanism 26 includes a first drive mechanism 26a and a second drive mechanism 26b. The first drive mechanism 26a is configured to move the holding pipette 16a to make the three-dimensional position of the holding pipette 16a variable. The second drive mechanism 26b is configured to move the injection pipette 16b to make the three-dimensional position of the injection pipette 16b variable. The three-dimensional position of each of the holding pipette 16a and the manipulator 16 is controllable independently of each other.

The lens drive mechanism 28 drives the variable focus lens 22 and changes the refractive power of the variable focus lens 22. The lens drive mechanism 28 changes the effective working distance in the combination of the objective lens 20 and the variable focus lens 22 by changing the refractive power of the variable focus lens 22. The effective working distance is a distance from the leading end of the objective lens 20 to the focal position of the observation light 42 and is a distance from the leading end of the objective lens 20 to the focal plane where the image captured by the imaging apparatus 24 is focused.

The control apparatus 30 controls the overall operation of the manipulation system 10. The hardware of the control apparatus 30 can be implemented by elements or mechanical devices such as a processor such as a computer's central processing unit (CPU) or graphics processing unit (GPU) and memory such as read only memory (ROM) or random access memory (RAM). The software of the control apparatus 30 is realized by a computer program, etc. The control apparatus 30 is comprised of, for example, of a general-purpose personal computer.

The display apparatus 32 includes a three-dimensional display apparatus 32a and a two-dimensional display apparatus 32b. The three-dimensional display apparatus 32a displays the three-dimensional positions of the sample 40 and the manipulator 16 in three dimensions. The three-dimensional display apparatus 32a is, for example, a hologram display such as Looking Glass and is a display apparatus that enables stereoscopic vision without using 3D glasses, etc. A computer graphic (CG) image that simulates the sample 40 and the manipulator 16 is displayed on the three-dimensional display apparatus 32a. The three-dimensional display apparatus 32a may be a head-mounted virtual reality (VR) display apparatus.

The two-dimensional display apparatus 32b is a liquid crystal display, etc. and displays an image captured by the imaging apparatus 24 in real time. The two-dimensional display apparatus 32b may display the three-dimensional positions of the sample 40 and the manipulator 16 and may display a rendered image generated by perspective projection of the sample 40 and the manipulator 16, mapped to a virtual space, onto an arbitrary observation surface.

The input apparatus 34 is an apparatus for providing an input to the control apparatus 30 and to manipulate the manipulator 16. A mouse, a keyboard, etc. can be used as a means to provide an input to the control apparatus 30. A joystick, etc. can be used to control the manipulator 16. By using the input apparatus 34 such as a joystick, the leading end position of the manipulator 16 can be moved on the order of micrometers, and the sample 40 can be manipulated with precision.

The force sensation presentation apparatus 36 is a means to manipulate the manipulator 16 and is configured to receive an input operation from the user for designating the position of the manipulator 16. The force sensation presentation apparatus 36 has multi-joint arms 36a and is configured so that a leading end 36b of the multi-joint arms 36a can be moved in three axes of XYZ. The user performs an input operation for specifying the three-dimensional position of the manipulator 16 by gripping and moving the leading end 36b of the multi-joint arms 36a. The force sensation presentation apparatus 36 includes, for example, a sensor for identifying the position of the leading end 36b of the multi-joint arms 36a and transmits position information based on the position of the leading end 36b of the multi-joint arm 36a to the control apparatus 30.

The force sensation presentation apparatus 36 is also a force sensation presentation means for presenting a force sensation during cell manipulation to the user. The force sensation presentation apparatus 36 is configured so that a force sensation in three axes of XYZ can be presented at the leading end 36b of the multi-joint arms 36a. The force sensation presentation apparatus 36 has an actuator for applying a reaction force to the multi-joint arms 36a and controls the operation of the actuator based on force information transmitted from the control apparatus 30.

In the embodiment, the input apparatus 34 such as a joystick is used to manipulate the holding pipette 16a, and the force sensation presentation apparatus 36 is used to manipulate the injection pipette 16b. The force sensation presentation apparatus 36 may be used to manipulate the holding pipette 16a.

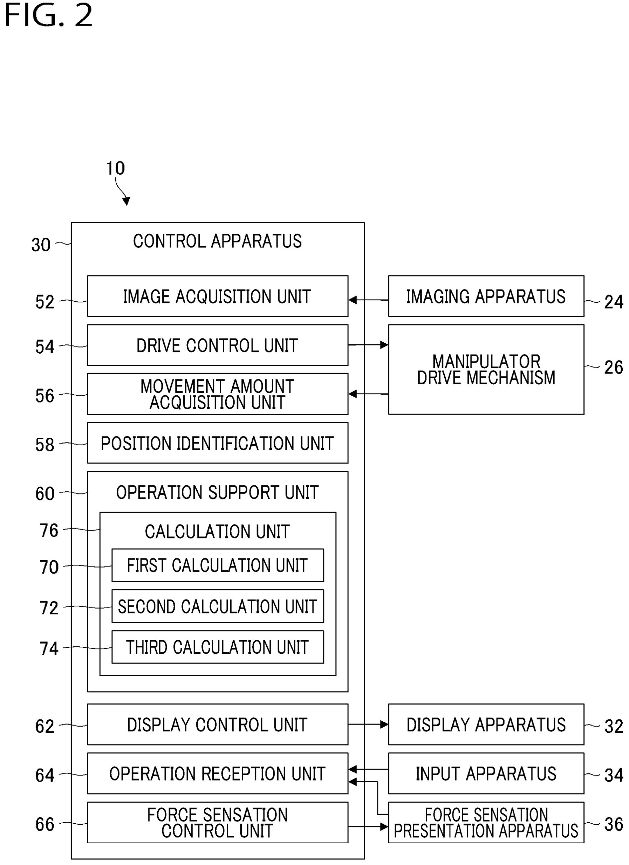

Fig. 2 is a block diagram schematically showing a functional configuration of a control apparatus 30 according to the first embodiment. The control apparatus 30 includes an image acquisition unit 52, a drive control unit 54, a movement amount acquisition unit 56, a position identification unit 58, an operation support unit 60, a display control unit 62, an operation reception unit 64, and a force sensation control unit 66.

The image acquisition unit 52 acquires an image captured by the imaging apparatus 24. The image acquired by the image acquisition unit 52 includes, for example, the sample 40 and a leading end of the manipulator 16 (a leading end 38a of the holding pipette 16a or a leading end 38b of the injection pipette 16b) that are imaged through the objective lens 20.

The drive control unit 54 controls the operation of the manipulator drive mechanism 26 to move the manipulator 16. The drive control unit 54 controls the operation of the manipulator drive mechanism 26 according to an input operation from the user through the input apparatus 34 and the force sensation presentation apparatus 36. For example, the drive control unit 54 controls the operation of the first drive mechanism 26a to move the holding pipette 16a according to an input operation entered through the input apparatus 34. For example, the drive control unit 54 controls the operation of the second drive mechanism 26b to move the injection pipette 16b according to an input operation entered through the force sensation presentation apparatus 36.

The movement amount acquisition unit 56 acquires a movement amount ΔPm of the manipulator 16 in a three-dimensional direction. The movement amount acquisition unit 56 acquires, for example, the rotation angle of the motor of the manipulator drive mechanism 26 and the amount of driving the actuator, and calculates a movement amount ΔPm(t) of the manipulator 16 in the three-dimensional direction at the current time t based on the acquired rotation angle and amount of driving. The movement amount acquisition unit 56 acquires the movement amounts of the leading ends 38a and 38b of the holding pipette 16a and the manipulator 16, respectively. The movement amount acquisition unit 56 may acquire a coordinate value indicating the current position of the manipulator 16 in the three-dimensional direction and calculate the movement amount from the amount of change in the coordinate value.

The position identification unit 58 identifies the position Pc(t) = (xc(t), yc(t), zc(t)) of the sample 40 and the position Pm(t) = (xm(t), ym(t), zm(t)) of the manipulator 16 at the current time t based on the image acquired by the image acquisition unit 52. The position identification unit 58 identifies the sample 40 and the leading end of the manipulator 16 included in the image using image recognition technology, and identifies the three-dimensional positions of the sample 40 and the leading end of the manipulator 16 using a coordinate system based on an optical axis A of the objective lens 20. For example, the position identification unit 58 identifies the three-dimensional position of the sample 40 and the leading end of the manipulator 16 every predetermined time period (e.g., 40 milliseconds).

The position identification unit 58 may identify the current position Pm(t) of the manipulator 16 based on the movement amount ΔPm(t) of the manipulator 16 acquired by the movement amount acquisition unit 56. The position identification unit 58 may identify the current position Pm(t) of the manipulator 16 using an expression: Pm(t) = Pm(t-1) + ΔPm(t). The position identification unit 58 may, for example, identify the origin position of the leading end of the manipulator 16 based on the image acquired by the image acquisition unit 52 and identify the current position by integrating the movement amount acquired by the movement amount acquisition unit 56 to the origin position. The position identification unit 58 can identify the three-dimensional position using the method described in JP 2022-57695.

The operation support unit 60 generates operation support information for performing a predetermined operation on the sample 40 based on the position of the sample 40 and the movement amount of the manipulator 16. The predetermined operation is, for example, an operation to rotate the sample 40 held at the leading end 38a of the holding pipette 16a in a predetermined direction using the injection pipette 16b, which is an operation to rotate the sample 40 around the x or z axis.

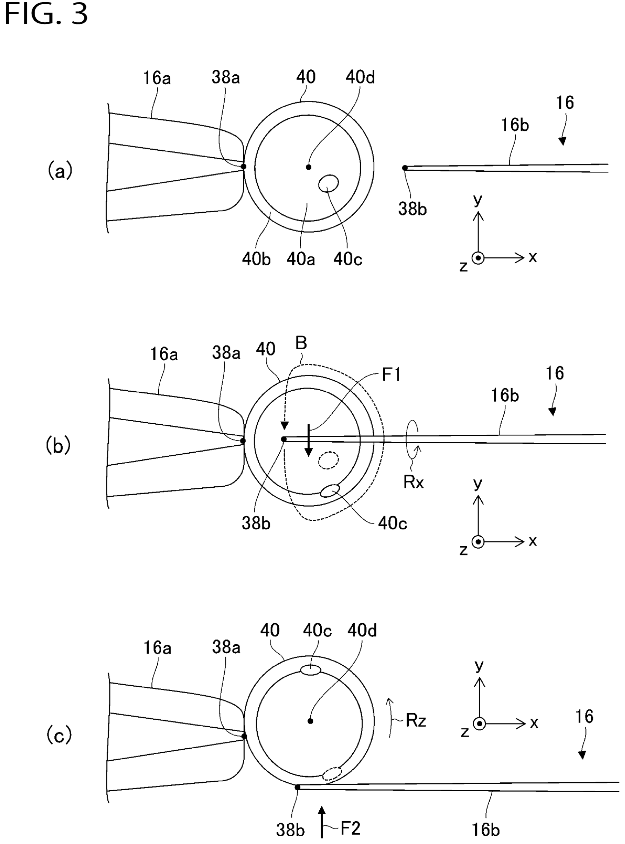

Figs. 3(a)-3(c) are diagrams schematically showing how the sample 40 is manipulated by the manipulator 16. Fig. 3(a) shows an operation of holding the sample 40. Fig. 3(b) and Fig. 3(c) show an operation of rotating the sample 40. Figs. 3(a)-3(c) correspond to images captured by the imaging apparatus 24 and show states in which the sample 40 is viewed in the z direction. In Figs. 3(a)-3(c), the direction of protrusion of the leading end 38a of the holding pipette 16a is shown as the +x direction.

In the holding operation in Fig. 3(a), the sample 40 is fixed by bringing the leading end 38a of the holding pipette 16a in close proximity to the sample 40 and sucking the sample 40 by the leading end 38a of the holding pipette 16a. The sample 40 is in contact with the leading end 38a of the holding pipette 16a in the x direction, and the sample 40 is held by a holding force in the x direction (-x direction) caused by the suction. An example of the sample 40 is a mature human or animal egg cell having a central cytoplasm 40a, a zona pellucida 40b surrounding the cytoplasm 40a, and a polar body 40c at a certain location between the cytoplasm 40a and zona pellucida 40b.

Fig. 3(b) shows an operation of rotating the sample 40 around the x-axis by using the injection pipette 16b so as to adjust the position of the polar body 40c. By rubbing the side of the sample 40 with the injection pipette 16b, the sample 40 can be rotated around the x-axis. For example, by moving the injection pipette 16b downward (-y direction) so as to slightly touch the side of the sample 40 in the +z direction (the front side of the paper in Fig. 3(b)) and applying a downward (-y direction) force F1 to the sample 40, the sample 40 can be rotated around the x-axis. The sample 40 can also be rotated in the opposite direction around the x-axis by moving the injection pipette 16b upward (+y direction).

For example, the rotation operation shown in Fig. 3(b) may be performed multiple times until the polar body 40c of the sample 40 is at the desired position. When repeating the rotation operation, it is necessary to pull out the injection pipette 16b to a position where the injection pipette 16b does not come into contact with the side of the sample 40, then move the injection pipette 16b upward (+y direction), and then bring the injection pipette 16b closer to the sample 40. For example, by moving the leading end 38b of the injection pipette 16b along an operation trajectory B indicated by a dashed line, multiple rotation operations can be performed in succession.

The rotational operation shown in Fig. 3(b) requires an operation of bringing the injection pipette 16b to slightly come into contact with the sample 40 and rub the sample 40, and the three-dimensional position of the injection pipette 16b needs to be precisely controlled according to the position and size of the sample 40. Therefore, the operation of rotating the sample 40 around the x-axis is highly challenging and requires a skillful technique for the rotation operation while minimizing cell damage.

Fig. 3(c) shows an operation of rotating the sample 40 around the z-axis by using the injection pipette 16b so as to adjust the position of the polar body 40c. By pressing the sample 40 with the injection pipette 16b, the sample 40 can be rotated around the z-axis. For example, when the injection pipette 16b is pressed against the sample 40 in the +y direction, a force F2 in the +y direction is applied to the sample 40, and the central position 40d of the sample 40 is shifted in the +y direction. The sample 40 can be rotated around the z-axis by the suction force exerted on the sample 40 by the holding pipette 16a at this time. The rotation operation shown in Fig. 3(c) is less difficult than the rotation operation shown in Fig. 3(b).

The operation support unit 60, for example, generates operation support information to support the rotational operation around the x-axis shown in Fig. 3(b). As the operation support information, the operation support unit 60 calculates a target movement amount ΔP, which is the next movement amount of the manipulator 16 (e.g., the injection pipette 16b). The user can achieve a predetermined operation (e.g., rotation operation around x-axis) on the sample 40 by moving the manipulator 16 (e.g., injection pipette 16b) according to the target movement amount ΔP calculated by the operation support unit 60.

The operation support unit 60 includes a calculation unit 76. The calculation unit 76 includes a first calculation unit 70, a second calculation unit 72, and a third calculation unit 74.

The first calculation unit 70 uses the position Pc(t) of the sample 40 and the movement amount ΔPm(t) of the manipulator 16 as input and calculates a first movement amount ΔP1(t+1) of the manipulator 16 for performing a predetermined operation on the sample 40. The first movement amount ΔP1(t+1) corresponds to the movement amount of the manipulator 16 at the next time (t+1) necessary to properly manipulate the sample 40 in a situation where the position of the sample 40 changes due to the manipulator 16 (e.g., injection pipette 16b) coming into contact with the sample 40. For example, by considering the relationship between the movement amount of the manipulator 16 and the position of the sample 40, the first calculation unit 70 can successively calculate the first movement amount fed back to approach the ideal operation when a deviation from the ideal operation occurs.

The first calculation unit 70 may calculate the first movement amount using, as input, time-series data in which the position of the sample 40 and the movement amount of the manipulator 16 (e.g., injection pipette 16b) are associated with each other in time series. The time-series data of the position of the sample 40 used by the first calculation unit 70 as input is past position data identified by the position identification unit 58. The time-series data of the movement amount of the manipulator 16 used by the first calculation unit 70 as input is past movement amount data acquired by the movement amount acquisition unit 56. The first calculation unit 70 uses as input the time-series data of the position of the sample 40 and the movement amount of the manipulator 16 during the most recent predetermined period (e.g., one or two seconds). In other words, the first calculation unit 70 calculates the first movement amount ΔP1(t+1) using the position Pc(t-n) of the sample 40 during a predetermined period (from t-n to t), ..., Pc(t), and the movement amount ΔPm(t-n) of the manipulator 16 as input. As an example, when the position of the sample 40 and the movement amount of the manipulator 16 are acquired at 40 millisecond intervals, time-series data of 50 (i.e., n = 49) positions of the sample 40 and movement amounts of the manipulator 16 acquired in the last two seconds are used. By using the time-series data as input, the operation process (i.e., time series) up to the present can be taken into consideration, and the movement amount can be calculated sequentially with appropriate feedback to ensure an ideal operation.

The first calculation unit 70 may calculate the first movement amount by using a trained model that has undergone machine-learning using teaching data in which the position of the sample 40 and the movement amount of the manipulator 16 are associated with each other in time series, which are acquired when a skilled operator performs the predetermined operation. The trained model can be generated by supervised learning by using the time-series data of the position of the sample 40 and the movement amount of the manipulator 16 during the predetermined period as input and the movement amount of the manipulator 16 immediately after the predetermined period as output. As a model for machine learning, a recurrent neural network (RNN) suitable for handling time-series data, and the like can be used, for example, a long short-term memory (LSTM) can be used.

The second calculation unit 72 uses the position Pm(t) of the manipulator 16 and the movement amount ΔPm(t) of the manipulator 16 as input and calculates a second movement amount ΔP2(t+1) of the manipulator 16 for guiding the manipulator 16 to a reference trajectory set around the sample 40. In this case, the reference trajectory corresponds to an ideal trajectory of the leading end of the manipulator 16 when performing a predetermined operation on the sample 40, for example, corresponds to an operation trajectory B shown in Fig. 3(b).

The reference trajectory can be determined based on the distribution of positions of the manipulator 16 acquired when a skilled operator performs a predetermined operation. For example, the reference trajectory can be determined by acquiring position data of the manipulator 16 when a skilled operator performs a predetermined operation and calculating a probability density function representing the distribution of the acquired positions of the manipulator 16. As an example, the parameters of the probability density function (e.g., the mean and standard deviation of each of multiple Gaussian distributions) can be determined by cluster analyzing the position data using a machine learning model such as a Gaussian mixture model (GMM).

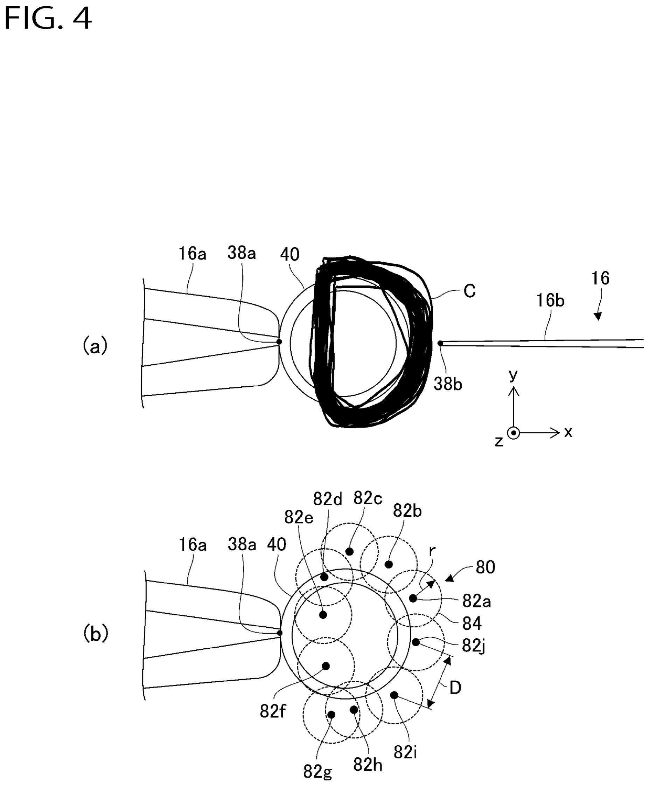

Fig. 4(a) shows an example of an operation trajectory C of the manipulator 16 when a skilled operator performs a predetermined operation. The operation trajectory C indicated by a bold line in Fig. 4(a) shows the trajectory of the leading end 38b of the injection pipette 16b when the operation of rotating the sample 40 around the x-axis is repeated. Fig. 4(b) shows an example of a reference trajectory 80 set around the sample 40. The reference trajectory 80 in Fig. 4(b) is a reference trajectory generated by machine learning by using the operation trajectory C in Fig. 4(a) as input data. The reference trajectory 80 is defined by the average positions 82a, 82b, 82c, 82d, 82e, 82f, 82g, 82h, 82i, 82j of ten Gaussian distributions. The number of Gaussian distributions defining the reference trajectory 80 is not particularly limited and can be adjusted appropriately according to the range and variation occupied by the operation trajectory C used for input data.

Fig. 4(b) further shows a neighboring area 84 indicated by a dashed line of a radius r centered at each of the ten average positions 82a-82j. The neighboring area 84 is a range that can be considered ideal for performing the predetermined operation. The radius r of the neighboring area 84 is set such that, for example, ten neighboring areas 84 are contiguous and is set such that, for example, the radius r is equal to or greater than half the interval D between adjacent average positions (that is, r ≥ D/2). The radius r of the neighboring area 84 is set to be smaller than the size of the sample 40 and is set to be equal to or less than half the diameter of the sample 40. An example of the radius r of the neighboring area 84 is 30 µm. Instead of setting the radius r of the neighboring area 84 to a fixed value, the value of the standard deviation of each Gaussian distribution may be used.

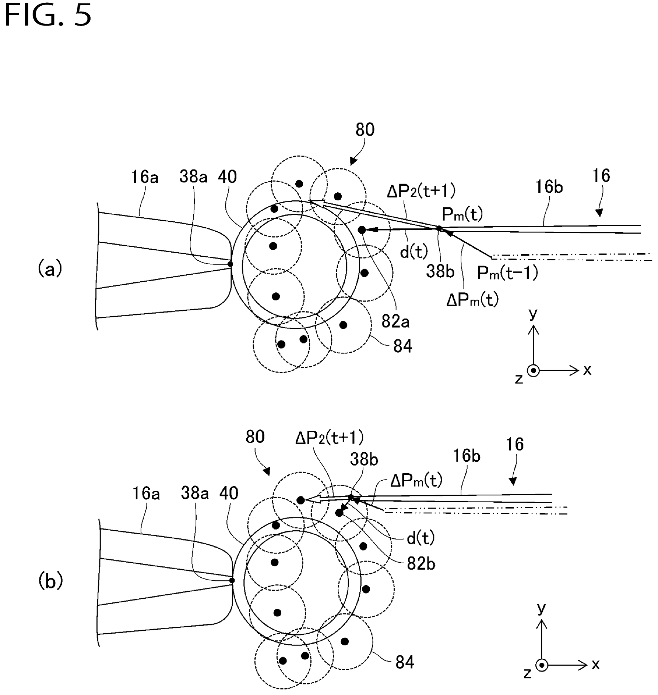

The second calculation unit 72 calculates a distance vector d(t) from the current position Pm(t) of the manipulator 16 to the reference trajectory 80. The second calculation unit 72 adds the movement amount ΔPm(t) of the manipulator 16 to the distance vector d(t) and calculates the second movement amount ΔP2(t+1) = d(t) + ΔPm(t). Figs. 5(a) and 5(b) schematically show how the second movement amount ΔP2(t+1) is calculated.

Fig. 5(a) shows a case where the leading end of the manipulator 16 (leading end 38b of the injection pipette 16b) is away from the reference trajectory 80 (or neighboring area 84). The second calculation unit 72 calculates a distance vector d(t) from the current position Pm(t) of the manipulator 16 to the reference trajectory 80. The closest average position (82a in the case of Fig. 5(a)) from the current position Pm(t) of the manipulator 16 is used as the position of the reference trajectory 80. The distance vector d(t) is a vector in a direction that guides the manipulator 16 toward the reference trajectory 80. The second calculation unit 72 adds the immediately preceding movement amount ΔPm(t) of the manipulator 16 to the distance vector d(t) and calculates the second movement amount ΔP2(t+1). By adding the immediately preceding movement amount ΔPm(t) of the manipulator 16, sudden changes in the movement direction of the manipulator 16 are suppressed, and the second movement amount ΔP2(t+1) for the next time for moving the manipulator 16 smoothly can be calculated. The second movement amount ΔP2(t+1) is in the direction that brings the manipulator 16 closer to the reference trajectory 80 when the manipulator 16 is away from the sample 40.

Fig. 5(b) shows a case where the leading end of the manipulator 16 (leading end 38b of the injection pipette 16b) is located in the vicinity (or within the neighboring area 84) of the reference trajectory 80. In the same way as in Fig. 5(a), the second calculation unit 72 calculates the distance vector d(t) from the current position Pm(t) of the manipulator 16 to the reference trajectory 80. The closest average position (82b in the case of Fig. 5(b)) from the current position Pm(t) of the manipulator 16 is used as the position of the reference trajectory 80. The second calculation unit 72 adds the immediately preceding movement amount ΔPm(t) of the manipulator 16 to the distance vector d(t) and calculates the second movement amount ΔP2(t+1). By adding the immediately preceding movement amount ΔPm(t) of the manipulator 16, sudden changes in the movement direction of the manipulator 16 are suppressed, and the second movement amount ΔP2(t+1) for the next time for moving the manipulator 16 smoothly can be calculated. The second movement amount ΔP2(t+1) is in the direction in which the manipulator 16 is moved along the reference trajectory 80 when the manipulator 16 is located in the vicinity of the reference trajectory 80.

The third calculation unit 74 calculates the target movement amount ΔP(t+1) of the manipulator 16 at the next time (t+1) by using the first movement amount ΔP1(t+1) calculated by the first calculation unit 70 and the second movement amount ΔP2(t+1) calculated by the second calculation unit 72. The third calculation unit 74 calculates the target movement amount ΔP(t+1) by weight-averaging the first movement amount ΔP1(t+1) and the second movement amount ΔP2(t+1) by using a weight coefficient α according to the distance d (size of the distance vector d(t)) from the current position Pm(t) of the manipulator 16 to the reference trajectory 80. The target movement amount can be expressed as ΔP(t+1) = α·ΔP1(t+1) + β(1-α)·ΔP2(t+2) using a scaling factor β. The weight coefficient α can be set such that the contribution of the first movement amount ΔP1(t+1) is greater when the distance d is small (i.e., close to the sample 40) and that the contribution of the second movement amount ΔP2(t+1) is greater when the distance d is large (i.e., far from the sample 40).

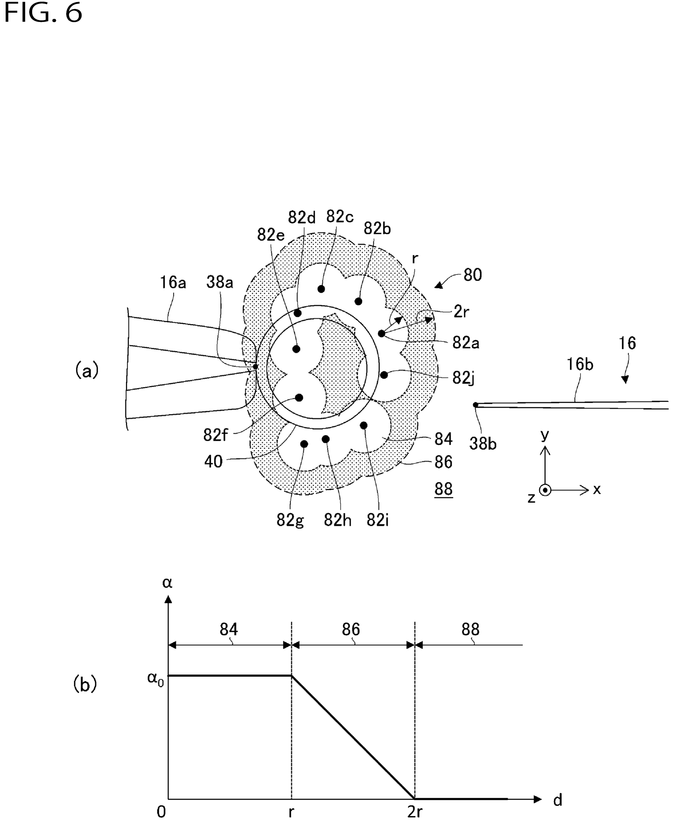

Figs. 6(a) and 6(B) are diagrams schematically showing an example of setting the weight coefficient α for calculating the target movement amount ΔP(t+1). Fig. 6(a) shows a neighboring area 84, an adjacent area 86, and an external area 88, which are set according to the distance from the reference trajectory 80. Fig. 6(b) is a graph showing the weight coefficient α set in each of the neighboring area 84, the adjacent area 86, and the external area 88. The neighboring area 84 is an area whose distance from the reference trajectory 80 is within a radius r (e.g., r = 30 µm). In the neighboring area 84, the weight coefficient α is set to a constant value α₀ (e.g., 0.8) regardless of the distance d. The adjacent area 86 is an area where the distance from the reference trajectory 80 is greater than the radius r and within a radius 2r (e.g., 2r = 60 µm). In the adjacent area 86, the weight factor α is set to decrease as the distance d increases and is expressed as α = α₀·(2-d/r). The external area 88 is an area whose distance from the reference trajectory 80 is greater than the radius 2r. In the external area 88, the weight coefficient α = 0 is set regardless of the distance d.

The third calculation unit 74 calculates the target position P(t+1) of the manipulator 16 at the next time (t+1) by using the calculated target movement ΔP(t+1). The target position P(t+1) is the current position Pm(t) of the manipulator 16 plus the target movement amount ΔP(t+1) and is expressed as P(t+1) = Pm(t) + ΔP(t+1).

The display control unit 62 controls the display on a display apparatus 32. The display control unit 62 generates and displays on the display apparatus 32 an image in which operation support information for a predetermined operation is superimposed on the image acquired by the image acquisition unit 52. The operation support information displayed on the display apparatus 32 includes the target movement amount ΔP(t+1) calculated by the operation support unit 60, the target position P(t+1), the reference trajectory 80, etc.

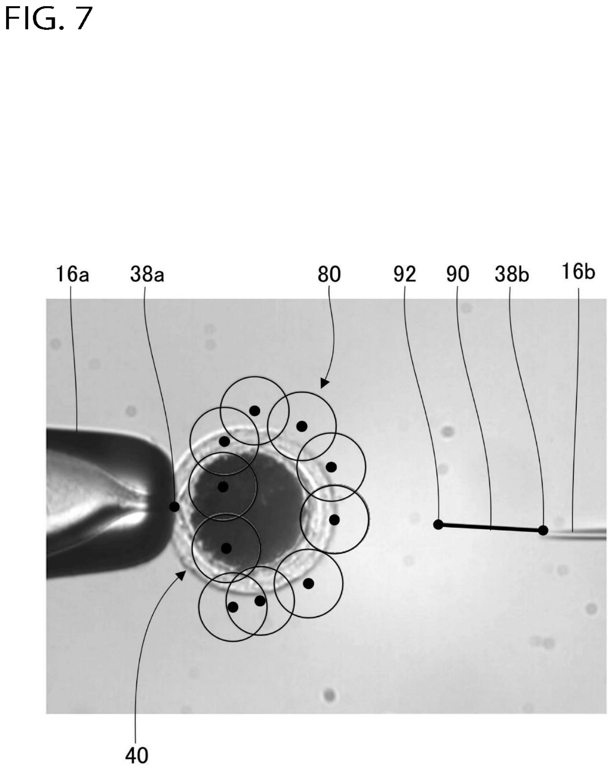

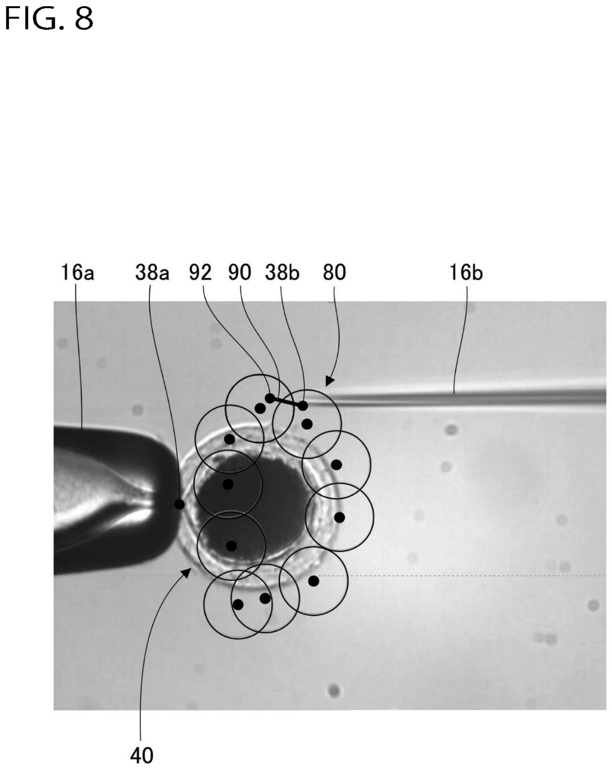

Figs. 7 and 8 are diagrams showing an example of the operation support information displayed on the display apparatus 32. Fig. 7 is an example screen when the leading end 38b of the injection pipette 16b is away from the sample 40 held at the leading end 38a of the holding pipette 16a. Fig. 8 is an example screen when the leading end 38b of the injection pipette 16b is in the vicinity of the sample 40. As shown in Figs. 7 and 8, the reference trajectory 80 is displayed while being superimposed in the vicinity of the sample 40, and a straight line 90 indicating the target movement amount ΔP(t+1) extending from the leading end 38b of the injection pipette 16b and a point 92 indicating the target position P(t+1) are also displayed while being superimposed.

The operation reception unit 64 receives an input operation for specifying the position of the manipulator 16 from the input apparatus 34 or the force sensation presentation apparatus 36. The drive control unit 54 moves the manipulator 16 based on the input operation information received at the operation reception unit 64.

The force sensation control unit 66 generates force information indicating the direction and magnitude of the force sensation to be presented to the user through the force sensation presentation apparatus 36. The force sensation control unit 66 generates force information in accordance with the target movement amount ΔP(t+1) calculated by the operation support unit 60. The force sensation control unit 66 generates force information in the same direction as the target movement amount ΔP (t+1). The force sensation control unit 66 may generate force information according to the magnitude of the target movement amount ΔP(t+1). For example, if the magnitude of the target movement amount ΔP(t+1) is equal to or greater than a predetermined value (e.g., 10 µm), the force sensation control unit 66 generates force information in which the magnitude of the force has a constant value f₀ regardless of the magnitude of the target movement amount ΔP(t+1). For example, if the magnitude of the target movement amount ΔP(t+1) is less than the predetermined value (e.g., 10 µm), the force sensation control unit 66 generates force information in which the magnitude of the force is variable (e.g., proportional) according to the magnitude of the target movement amount ΔP(t+1).

In the display example in Fig. 7, since the leading end 38b of the injection pipette 16b is located in the external area 88 (see Fig. 6(a)), the target movement amount ΔP(t+1) is determined by the second movement amount ΔP2(t+1). In the display example in Fig. 7, since the target movement amount ΔP(t+1) is relatively large, a force sensation that mainly has a constant value f₀ is presented. When the target movement amount ΔP(t+1) is large, it is not so important to strictly control the movement amount of the manipulator 16, and the operation can be a proper operation as long as the movement direction of the manipulator 16 is correct. In such a situation, by setting the magnitude of the force to be presented to have a constant value f₀, the user can operate the manipulator 16 without being particularly conscious of the movement amount (or movement speed).

In the display example in Fig. 8, since the leading end 38b of the injection pipette 16b is located in the neighboring area 84 (see Fig. 6(a)), the target movement amount ΔP(t+1) is mainly determined by the first movement amount ΔP1(t+1). In the display example in Fig. 8, since the target movement amount ΔP(t+1) is relatively small, a force sensation is presented in which the magnitude of the force is variable in proportion to the magnitude of the target movement amount ΔP(t+1). When the target movement amount ΔP(t+1) is small, it can be important to strictly control the movement amount of manipulator 16. In such a situation, setting the magnitude of the force sensation to be variable makes the user more aware of the movement amount (or movement speed) and allows the manipulator 16 to be operated more precisely.

According to the present embodiment, the user can grasp the target movement amount ΔP(t+1) through visual and force sensations and can properly operate the manipulator 16 according to the support through the visual and force sensations. In the display example in Fig. 7, the operation to bring the injection pipette 16b closer to the reference trajectory 80 can be supported. In the display example in Fig. 8, the operation of rotating and moving the injection pipette 16b along the reference trajectory 80 can be supported. As a result, the accuracy of the operation of rotating the sample 40 can be improved.

If the user wishes to stop the operation, the operation can be stopped at any time and the injection pipette 16b can be moved (e.g., retracted) to any position. If the user then wishes to resume the operation, the injection pipette 16b can be guided to move closer to the reference trajectory 80 and then rotate and move along the reference trajectory 80. As a result, support in a proper operation is possible for the user to perform a predetermined operation (for example, a rotation operation) on the sample 40 without impairing the degree of freedom of operation at the will of the user.

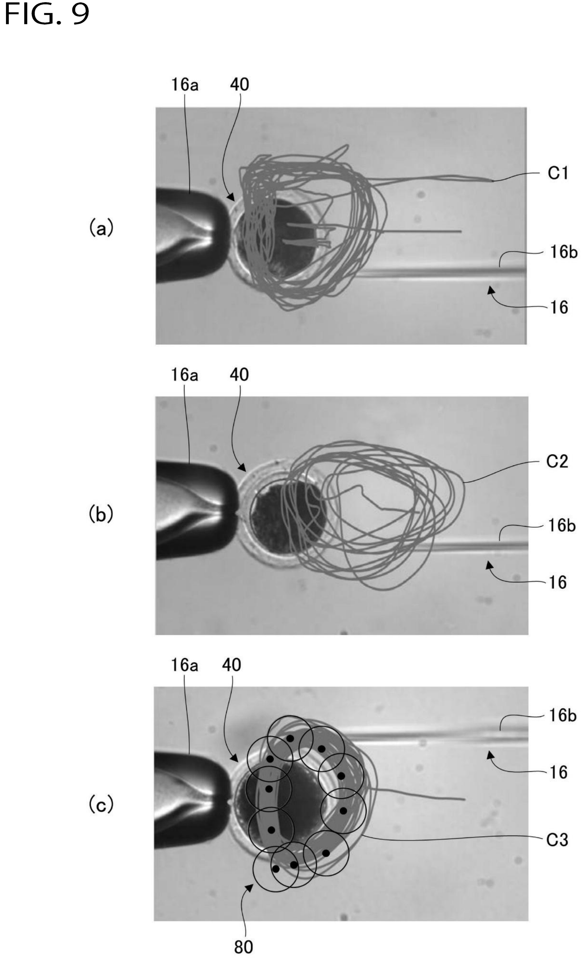

Figs. 9(a) to 9(c) are diagrams showing an example of operation trajectories C1, C2, and C3 of the manipulator when a beginner performs a predetermined operation.

Fig. 9(a) shows a comparative example when there is no visual or force support using operation support information. The operator performs an operation by following the operation trajectory C made by the skilled operator (see Fig. 4(a)); however, due to the lack of support, an operation trajectory C1 is not stable, and a zigzag-shaped trajectory is observed. Further, despite the fact that the predetermined operation is being performed, the rotation of the sample 40 may fail, causing the injection pipette 16b to collide with the sample 40 and damaging the sample 40.

Fig. 9(b) shows a comparative example in a case where there is support with visual and force sensations using operation support information, which is different from the case in the embodiment. In this comparative example, the operation support information is generated using a trained model where the time-series data of the position of the sample 40 and the position of the operation trajectory C made by the skilled operator is used as input and the target position of the injection pipette 16b at the next time is used as output. Therefore, this comparative example differs from the embodiment in that the movement amount of the operation trajectory C made by the skilled operator is not used as input, and differs from the embodiment in that the first and second movement amounts are not combined.

In Fig. 9(b), an operation trajectory C2 is relatively stable and does not show many zigzag-shaped trajectories since support through the visual and force sensations is provided. However, the operator is often guided to a target position that deviates from the operation trajectory C made by the skilled operator, resulting in failure to rotate the sample 40 or collision of the injection pipette 16b with the sample 40.

Fig. 9(c) shows an exemplary embodiment in a case where there is support through the visual and force sensations using the operation support information according to the embodiment. An operation trajectory C3 is stable and does not show many zigzag-shaped trajectories since the support through the visual and force sensations is provided. Further, the fact that the operation trajectory C3 is concentrated in the vicinity of the reference trajectory 80 indicates that the position of the injection pipette 16b can be guided properly. According to the present exemplary embodiment, the time required for the operation of rotating the sample 40 was able to be shortened, causing almost no collisions of the injection pipette 16b with the sample 40. Therefore, it can be found that according to the present exemplary embodiment, the operation of rotating the sample 40 can be properly supported, and improved operation accuracy can be achieved.

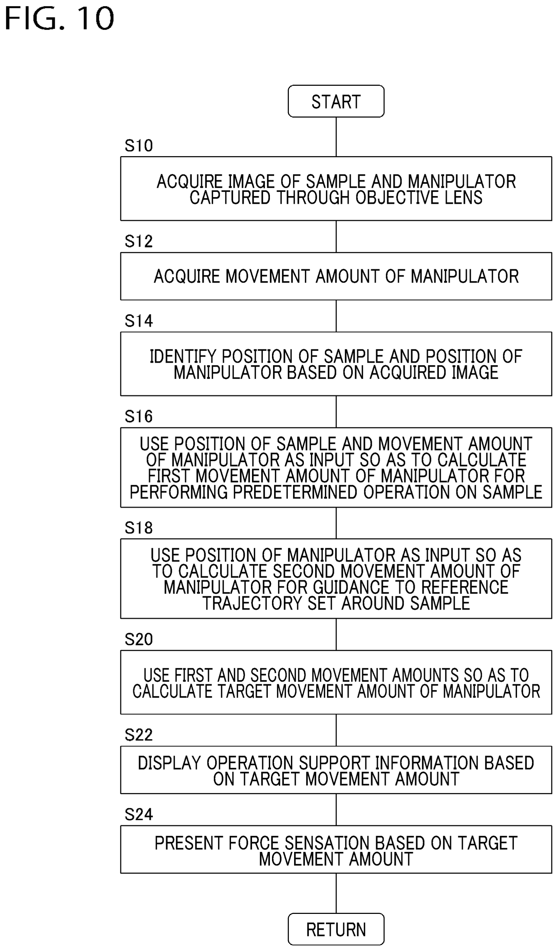

Fig. 10 is a flowchart showing an example of a manipulation method according to the first embodiment. The image acquisition unit 52 acquires an image of the sample 40 and the manipulator 16 captured through the objective lens 20 (S10). The movement amount acquisition unit 56 acquires the movement amount of the manipulator 16 (S12). The position identification unit 58 identifies the position of the sample 40 and the position of the manipulator 16 based on the acquired image (S14). The first calculation unit 70 uses the position of the sample 40 and the movement amount of the manipulator 16 as input and calculates a first movement amount of the manipulator 16 for performing the predetermined operation on the sample 40 (S16). The second calculation unit 72 uses the position of the manipulator 16 as input and calculates a second movement amount of the manipulator for guiding the manipulator 16 to the reference trajectory 80 set around the sample 40 (S18). The third calculation unit 74 uses the first and second movement amounts so as to calculate a target movement amount of the manipulator 16 (S20). The display control unit 62 displays operation support information that is based on the target movement amount on the display apparatus 32 (S22). The force sensation control unit 66 presents force sensation that is based on the target movement amount on the force sensation presentation apparatus 36 (S24).

### (Second embodiment)

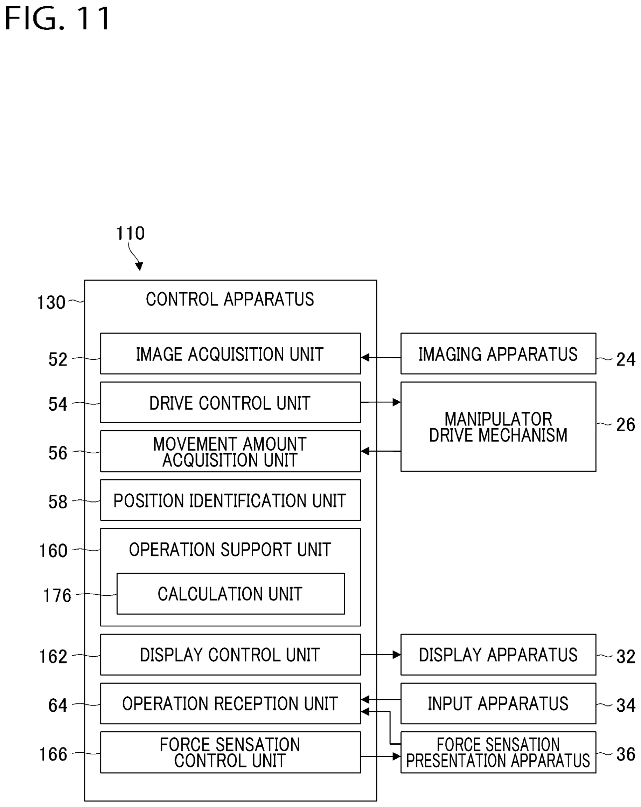

Fig. 11 is a block diagram schematically showing a functional configuration of a control apparatus 130 according to the second embodiment. The second embodiment differs from the first embodiment in that an operation support unit 160 includes a calculation unit 176. The second embodiment will be explained mainly on the differences from the first embodiment in the following, and explanations on common features will be omitted.

A manipulation system 110 according to the second embodiment is configured in the same manner as the first embodiment shown in Fig. 1 and includes a control apparatus 130 instead of a control apparatus 30. The control apparatus 130 is configured in the same manner as the control apparatus 30 shown in Fig. 2. However, instead of the operation support unit 60, the display control unit 62, and the force sensation control unit 66, the control apparatus 130 includes an operation support unit 160, a display control unit 162, and a force sensation control unit 66. The operation support unit 160 includes a calculation unit 176.

The calculation unit 176 uses the position Pc(t) of the sample 40 and the position Pm(t) of the manipulator 16 as input and calculates a target position P(t+1) of the manipulator 16 for performing the predetermined operation on the sample 40. The calculation unit 176 differs from the first calculation unit 70 according to the first embodiment in that the calculation unit 176 uses the position Pm(t) as input instead of the movement amount ΔPm(t) of the manipulator 16. The calculation unit 176 differs from the first calculation unit 70 according to the first embodiment in that the calculation unit 176 uses the target position P(t+1) as output instead of the target movement amount ΔP(t+1) of the manipulator 16.

The calculation unit 176 may calculate the target position using, as input, time-series data in which the position of the sample 40 and the position of the manipulator 16 are associated with each other in time series. For example, the calculation unit 176 uses as input the time-series data of the position of the sample 40 and the position of the manipulator 16 during the most recent predetermined period (e.g., one or two seconds). The calculation unit 176 calculates the target position P(t+1) using positions Pc(t-n), ..., Pc(t) of the sample 40 and positions Pm(t-n), ..., Pm(t) of the manipulator 16 during the predetermined period (from t-n to t). As an example, when the position of the sample 40 and the position of the manipulator 16 are acquired at 40 millisecond intervals, time-series data of 50 (i.e., n = 49) positions of the sample 40 and movement amounts of the manipulator 16 acquired in the last two seconds are used.

The calculation unit 176 may calculate the target position by using a trained model that has undergone machine-learning using teaching data in which the position of the sample 40 and the position of the manipulator 16 are associated with each other in time series, which are acquired when a skilled operator performs the predetermined operation. The trained model can be generated by supervised learning by using the time-series data of the position of the sample 40 and the position of the manipulator 16 during the predetermined period as input and the position of the manipulator 16 immediately after the predetermined period as output. As a model for machine learning, a recurrent neural network (RNN) suitable for handling time-series data, and the like can be used, for example, a long short-term memory (LSTM) can be used.

The calculation unit 176 may be configured to further calculate a reliability level R(t+1) of the target position P(t+1) of the manipulator 16. For example, by using a trained model that has machine-learned to output a distribution (e.g., a normal distribution) of the target position of the manipulator 16, the target position and reliability level of the manipulator 16 can be output. In this case, the average value (or maximum value) of the distribution output from the trained model can be used as the target position, and the standard deviation of the distribution output from the trained model can be used as the reliability level.

The calculation unit 176 calculates the target movement amount ΔP(t+1) of the manipulator 16 at the next time using the calculated target position P(t+1). The target movement amount ΔP(t+1) is the target position P(t+1) minus the current position Pm(t) of the manipulator 16 and is expressed as ΔP(t+1) = P(t+1) - Pm(t).

The calculation unit 176 may be configured to calculate the target position and reliability level of the manipulator 16 by combining multiple trained models or may use a so-called ensemble learning algorithm. The calculation unit 176 may use the average value of multiple output values output from the multiple trained models as the target position and use the standard deviation of the multiple output values as the reliability level. Each of the multiple trained models may have a network structure in which a training layer using an RNN or LSTM and a non-training layer are connected in parallel. By using a non-training layer, variations can be generated between the multiple output values from the multiple trained models, and the reliability level of the target position can be calculated based on the magnitude of the variations.

The display control unit 162 generates and displays on the display apparatus 32 an image in which operation support information for performing a predetermined operation is superimposed on the image acquired by the image acquisition unit 52. The operation support information displayed on the display apparatus 32 is the target position P(t+1) calculated by the operation support unit 160 and the reliability level R(t+1). The reliability level R(t+1) is displayed, for example, by a circle of radius R(t+1) centered at the target position P(t+1) (also referred to as confidence circle).

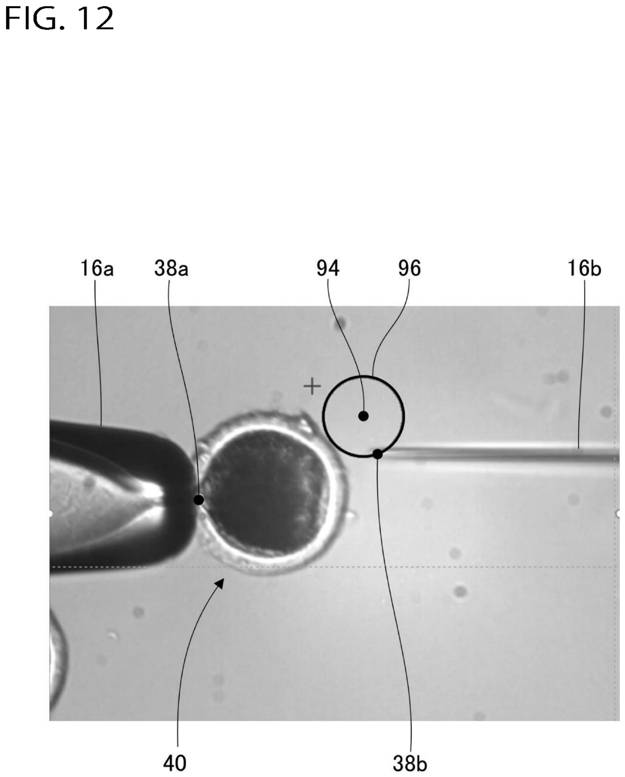

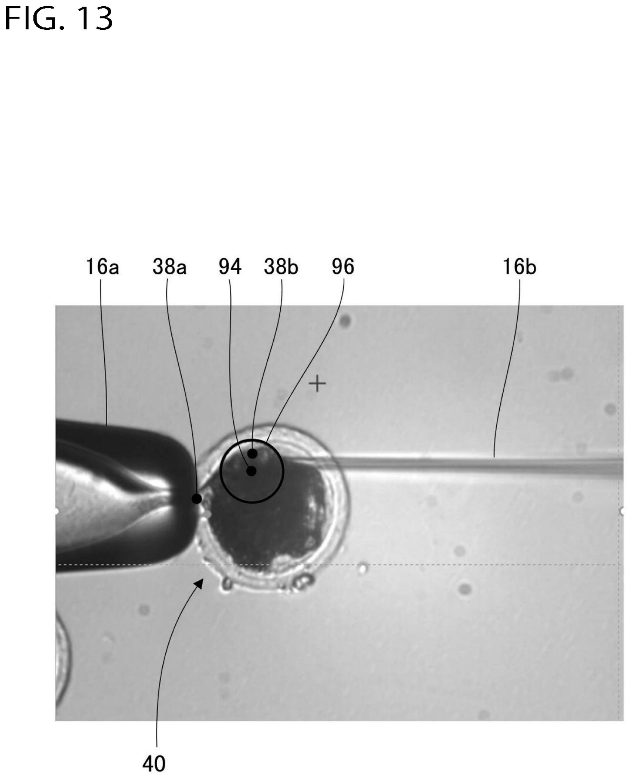

Figs. 12 and 13 are diagrams showing an example of the operation support information displayed on the display apparatus 32. Fig. 12 is an example screen when the leading end 38b of the injection pipette 16b is away from the sample 40 held at the leading end 38a of the holding pipette 16a. Fig. 13 is an example screen when the leading end 38b of the injection pipette 16b is in the vicinity of the sample 40. As shown in Figs. 12 and 13, a point 94 indicating the target position P(t+1) of the injection pipette 16b and a confidence circle 96 having a radius equal to the reliability level R(t+1) are displayed in a superimposed manner.

The force sensation control unit 66 generates force information indicating the direction and magnitude of the force sensation to be presented to the user through the force sensation presentation apparatus 36. The force sensation control unit 166 generates force information according to the target movement amount ΔP(t+1) calculated by the operation support unit 160. The force sensation control unit 166 generates force information in the same direction as the target movement amount ΔP(t+1). The force sensation control unit 66 may generate force information with a magnitude according to the target movement amount ΔP(t+1) and the reliability level R (t+1). For example, if the magnitude of the target movement amount ΔP(t+1) is larger than the reliability level R(t+1), that is, if the current position of the manipulator 16 is outside the confidence circle 96, the force sensation control unit 66 generates force information where the magnitude of the force has a constant value f₀ regardless of the magnitude of the target movement amount ΔP(t+1). For example, if the magnitude of the target movement amount ΔP(t+1) is smaller than the reliability level R(t+1), that is, if the current position of the manipulator 16 is inside the confidence circle 96, the force sensation control unit 66 generates force information where the magnitude of the force becomes variable according to the magnitude of the target movement amount ΔP(t+1).

In the display example in Fig. 12, the confidence circle 96 is displayed relatively large since the leading end 38b of the injection pipette 16b is relatively away from the sample 40. When the confidence circle 96 is large, the positional accuracy does not need to be strictly exact, and it is sufficient to operate the manipulator 16 toward a rough range indicated by the confidence circle 96.

In the display example in Fig. 13, the confidence circle 96 is displayed relatively small since the leading end 38b of the injection pipette 16b is close to the sample 40. When the confidence circle 96 is small, the positional accuracy needs to be exact, and the manipulator 16 is preferably operated with high precision toward a narrow range indicated by the confidence circle 96.

In the present embodiment, since the user can also grasp the target displacement ΔP(t+1) through visual and force sensations, the operation for rotating the sample 40 can be performed according to the support through the visual and force sensations. The user can grasp the required operation precision by the size of the confidence circle 96. For example, the user can roughly perform the operation without much concentration when the confidence circle 96 is large as in the display example in Fig. 12, while the user can concentrate and perform the operation precisely when the confidence circle 96 is small as in the display example in Fig. 13. The user can perform the well-balanced operation according to the required operation accuracy, thereby improving the operation accuracy.

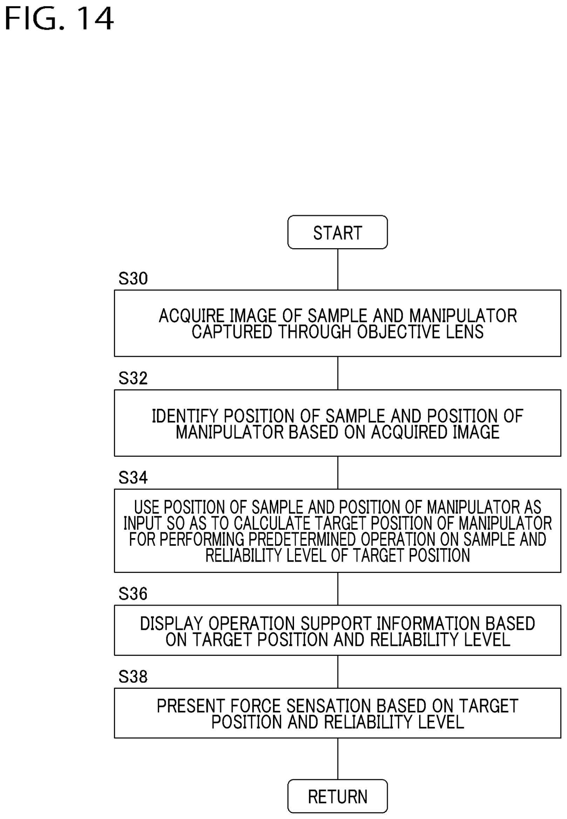

Fig. 14 is a flowchart showing an example of a manipulation method according to the second embodiment. The image acquisition unit 52 acquires an image of the sample 40 and the manipulator 16 captured through the objective lens 20 (S30). The position identification unit 58 identifies the position of the sample 40 and the position of the manipulator 16 based on the acquired image (S32). Using the position of the sample 40 and the position of the manipulator 16 as input, the calculation unit 176 calculates a target position of the manipulator 16 for performing a predetermined operation on the sample 40 and a reliability level of the target position (S34). The display control unit 162 displays operation support information based on the target position and the reliability level on the display apparatus 32 (S36). The force sensation control unit 66 presents the force sensation that is based on the target position and the reliability level on the force sensation presentation apparatus 36 (S24).

### (Third embodiment)

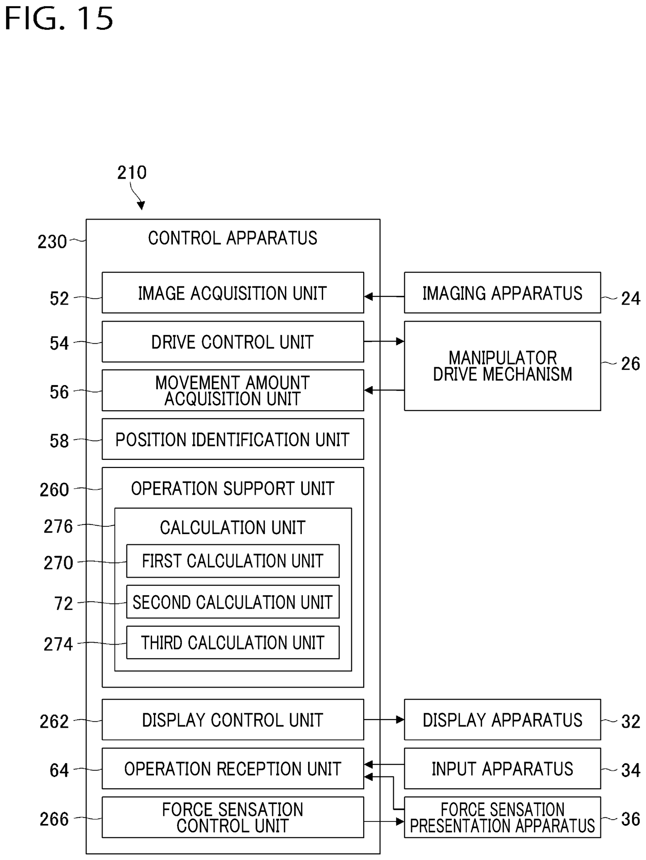

Fig. 15 is a block diagram schematically showing a functional configuration of a control apparatus 230 according to the third embodiment. The third embodiment differs from the first embodiment in that a first calculation unit 270 is configured to calculate the reliability level of the first movement amount. The third embodiment corresponds to a combination of the first embodiment and the features of the confidence circle according to the second embodiment. The third embodiment will be explained mainly on the differences from the first embodiment in the following, and explanations on common features will be omitted.

A manipulation system 210 according to the third embodiment is configured in the same manner as the first embodiment shown in Fig. 1 and includes a control apparatus 230 instead of a control apparatus 30. The control apparatus 230 is configured in the same manner as the control apparatus 30 shown in Fig. 2. However, instead of the operation support unit 60, the display control unit 62, and the force sensation control unit 66, the control apparatus 230 includes an operation support unit 260, a display control unit 262, and a force sensation control unit 266. The operation support unit 260 includes a calculation unit 276. The calculation unit 276 includes a first calculation unit 270, a second calculation unit 72, and a third calculation unit 274. The second calculation unit 72 can be configured in the same way as in the first embodiment.

The first calculation unit 270 is configured in the same way as in the first calculation unit 70 according to the first embodiment and is further configured to calculate the reliability level R1(t+1) of the first movement amount ΔP1(t+1) of the manipulator 16. For example, the first calculation unit 270 can output the first movement amount and reliability level of the manipulator 16 by using a trained model that has machine-learned to output a distribution (e.g., normal distribution) of the first movement amount ΔP1(t+1) of the manipulator 16. The first calculation unit 270 can calculate the reliability level using the same method as that in the second embodiment. For example, the first calculation unit 270 can set the average value (or maximum value) of the distribution output from the trained model as the first movement amount ΔP1(t+1) and the standard deviation of the distribution output from the trained model as the reliability level. The first calculation unit 270 may use an ensemble learning algorithm so as to calculate the first movement amount ΔP1(t+1) and the reliability level R1(t+1) of the manipulator 16.

The third calculation unit 274 is configured in the same way as in the third calculation unit 74 according to the first embodiment and is further configured to calculate the reliability level R1(t+1) of the target movement amount ΔP(t+1). The reliability level R(t+1) of the target movement amount ΔP(t+1) can be obtained, for example, by multiplying the reliability level R1(t+1) of the first movement amount ΔP1(t+1) by a weight coefficient α, which can be expressed as R(t+1)=α·R1(t+1).

The display control unit 262 generates and displays on the display apparatus 32 (e.g., two-dimensional display apparatus 32b) an image in which operation support information for performing a predetermined operation is superimposed on the image acquired by the image acquisition unit 52. The operation support information displayed on the display apparatus 32 includes the target movement amount ΔP(t+1) calculated by the operation support unit 60, the target position P(t+1), the reliability level R(t+1), the position of the reference trajectory 80, etc. The display control unit 262, for example, generates an image superimposed with a confidence circle having the target position P(t+1) as its center and the reliability level R(t+1) as its radius, and displays the image on the display apparatus 32. The confidence circle can be displayed in the same manner as the confidence circle 96 shown in Fig. 12 or Fig. 13.

The force sensation control unit 266 generates force information indicating the direction and magnitude of the force sensation to be presented to the user through the force sensation presentation apparatus 36. The force sensation control unit 266 generates force information according to the target movement amount ΔP(t+1) calculated by the operation support unit 260. The force sensation control unit 266 generates force information in the same direction as the target movement amount ΔP(t+1). The force sensation control unit 266 may generate force information with a magnitude according to the target movement amount ΔP(t+1) and the reliability level R (t+1). For example, if the magnitude of the target movement amount ΔP(t+1) is larger than the reliability level R(t+1), that is, if the current position of the manipulator 16 is outside the confidence circle 96, the force sensation control unit 266 generates force information where the magnitude of the force has a constant value f₀ regardless of the magnitude of the target movement amount ΔP(t+1). For example, if the magnitude of the target movement amount ΔP(t+1) is smaller than the reliability level R(t+1), that is, if the current position of the manipulator 16 is inside the confidence circle 96, the force sensation control unit 266 generates force information where the magnitude of the force becomes variable according to the magnitude of the target movement amount ΔP(t+1).

In the same way as in Fig. 7, since the leading end 38b of the injection pipette 16b is located in the external area 88 (see Fig. 6(a)), the weight coefficient α is equal to zero. In this case, the target movement amount ΔP(t+1) is determined by the second movement amount ΔP2(t+1), and the reliability level R(t+1) is equal to zero. As a result, the target movement amount ΔP(t+1) is larger than the reliability level R(t+1), and a force sensation is thus presented in which the magnitude of the force has a constant value f₀. The user can roughly operate the manipulator 16 without being particularly concerned about the magnitude of the force sensation.

In the same way as in Fig. 8, since the leading end 38b of the injection pipette 16b is located in the neighboring area 84 (see Fig. 6(a)), the weight coefficient α is equal to α₀ (e.g. 0.8). In this case, the target movement amount ΔP(t+1) is mainly determined by the first movement amount ΔP1(t+1), and the value of the reliability level R(t+1) is a non-zero value (e.g., about 10 µm to 30 µm). When the target movement amount ΔP(t+1) is smaller than the reliability level R(t+1), a force sensation of a magnitude corresponding to the target movement amount is presented, thus allowing the user to perform the operation in minute detail in response to changes in the force sensation. As a result, the user can operate the manipulator 16 more precisely according to the support through the visual and force sensations.

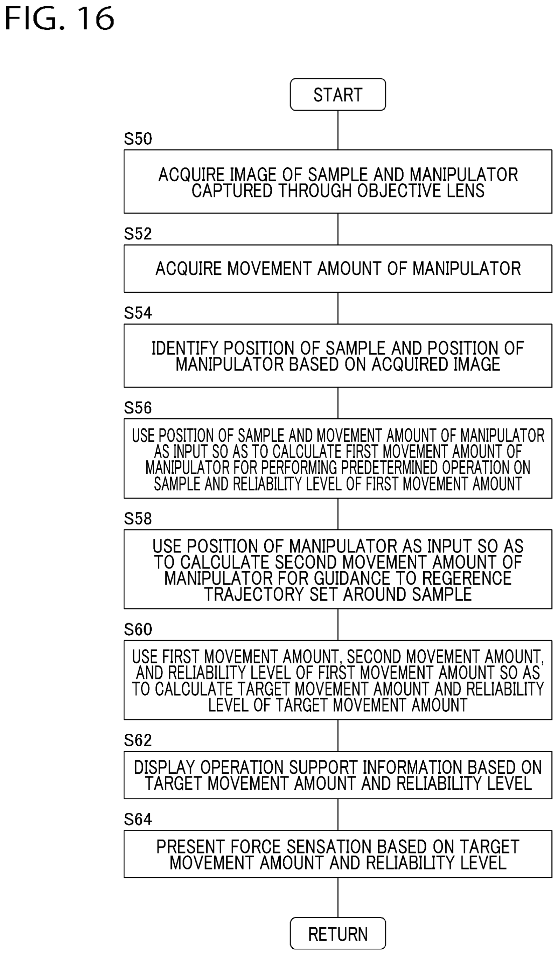

Fig. 16 is a flowchart showing an example of a manipulation method according to the third embodiment. The image acquisition unit 52 acquires an image of the sample 40 and the manipulator 16 captured through the objective lens 20 (S50). The movement amount acquisition unit 56 acquires the movement amount of the manipulator 16 (S52). The position identification unit 58 identifies the position of the sample 40 and the position of the manipulator 16 based on the acquired image (S54). The first calculation unit 270 uses the position of the sample 40 and the movement amount of the manipulator 16 as input and calculates a first movement amount of the manipulator 16 for performing the predetermined operation on the sample 40 and the reliability level of the first movement amount (S56). The second calculation unit 72 uses the position of the manipulator 16 as input and calculates a second movement amount of the manipulator for guiding the manipulator 16 to the reference trajectory 80 set around the sample 40 (S58). The third calculation unit 274 calculates the target movement amount of the manipulator 16 and the reliability level of the target movement amount using the first movement amount, the second movement amount, and the reliability level of the first movement amount (S60). The display control unit 262 displays operation support information based on the target movement amount and the reliability level on the display apparatus 32 (S62). The force sensation control unit 266 presents the force sensation that is based on the target position and the reliability level on the force sensation presentation apparatus 36 (S64).

### (Fourth embodiment)

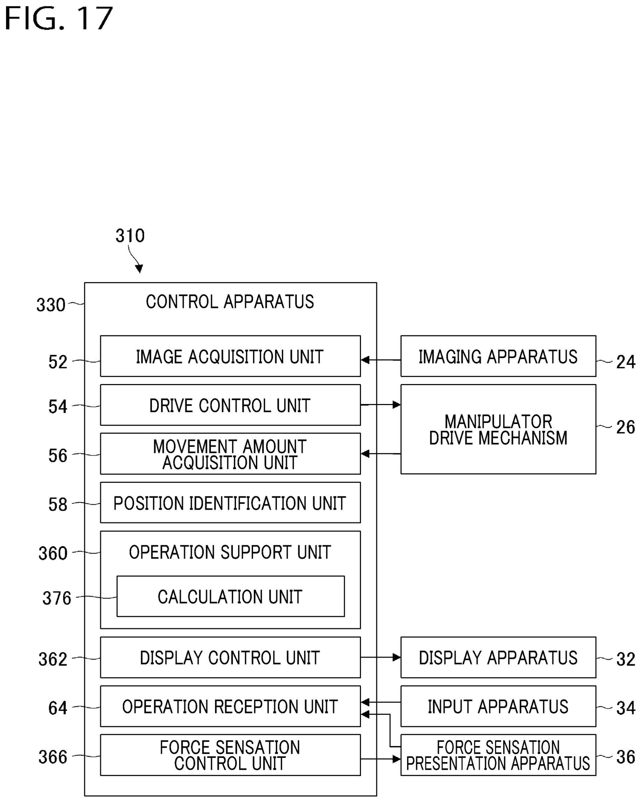

Fig. 17 is a block diagram schematically showing a functional configuration of a control apparatus 330 according to the fourth embodiment. The fourth embodiment differs from the above-stated embodiment in that an operation support unit 360 includes a calculation unit 376. The fourth embodiment will be explained mainly on the differences from the above-stated embodiment in the following, and explanations on common features will be omitted.

A manipulation system 310 according to the fourth embodiment is configured in the same manner as the first embodiment shown in Fig. 1 and includes a control apparatus 330 instead of a control apparatus 30. The control apparatus 330 is configured in the same manner as the control apparatus 30 shown in Fig. 2. However, instead of the operation support unit 60, the display control unit 62, and the force sensation control unit 66, the control apparatus 330 includes an operation support unit 360, a display control unit 362, and a force sensation control unit 366. The operation support unit 360 includes a calculation unit 376.

The calculation unit 376 calculates a target position P(t+1) or target movement amount ΔP(t+1) of the manipulator 16 for performing a predetermined operation on the sample 40 based on the position Pc(t) of the sample 40 and the position Pm(t) or movement amount ΔPm(t) of the manipulator 16. The calculation unit 376 may calculate both the target position P(t+1) and the target movement amount ΔP(t+1) of the manipulator 16.

The calculation unit 376 may be configured in the same way as in the first calculation unit 70 according to the first embodiment. The calculation unit 376 may calculate the target movement amount ΔP(t+1) of the manipulator 16 for performing the predetermined operation on the sample 40 by using the position Pc(t) of the sample 40 and the movement amount ΔPm(t) of the manipulator 16 as input. The calculation unit 376 may calculate the first movement amount ΔP1(t+1) calculated by the first calculation unit 70 as the target movement amount ΔP(t+1) (i.e., ΔP(t+1) = ΔP1(t+1)). In this case, the calculation unit 376 may calculate the target position P(t+1) = Pm(t) + ΔP(t+1) of the manipulator 16 by using the target movement amount ΔP(t+1) of the manipulator 16 and the current position Pm(t).

The calculation unit 376 may be configured in the same way as in the calculation unit 176 according to the second embodiment. The calculation unit 376 may use the position Pc(t) of the sample 40 and the position Pm(t) of the manipulator 16 as input and calculate the target position P(t+1) of the manipulator 16 for performing the predetermined operation on the sample 40. In this case, the calculation unit 376 may calculate the target movement ΔP(t+1) = P(t+1) - Pm(t) of the manipulator 16 by using the target position P(t+1) of the manipulator 16 and the current position Pm(t).

The display control unit 362 generates and displays on the display apparatus 32 (e.g., two-dimensional display apparatus 32b) an image in which operation support information for performing a predetermined operation is superimposed on the image acquired by the image acquisition unit 52. The operation support information displayed on the display apparatus 32 is the target position P(t+1) and the target movement amount ΔP(t+1) calculated by the operation support unit 360.

The force sensation control unit 366 generates force information indicating the direction and magnitude of the force sensation to be presented to the user through the force sensation presentation apparatus 36. The force sensation control unit 366 generates force information according to the target position P(t+1) or the target movement amount ΔP(t+1) calculated by the operation support unit 360. For example, the force sensation control unit 366 generates force information in the same direction as the target movement amount ΔP(t+1).

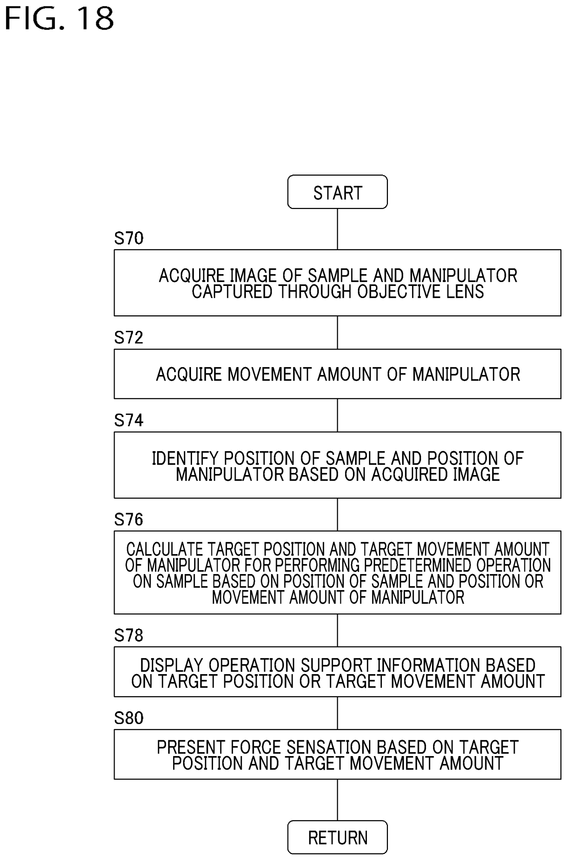

Fig. 18 is a flowchart showing an example of a manipulation method according to the fourth embodiment. The image acquisition unit 52 acquires an image of the sample 40 and the manipulator 16 captured through the objective lens 20 (S70). The movement amount acquisition unit 56 acquires the movement amount of the manipulator 16 (S72). The position identification unit 58 identifies the position of the sample 40 and the position of the manipulator 16 based on the acquired image (S74). The calculation unit 376 calculates a target position or target movement amount of the manipulator 16 for performing a predetermined operation on the sample 40 based on the position of the sample 40 and the position or movement amount of the manipulator 16 (S76). The display control unit 362 displays operation support information based on the target position or target movement amount on the display apparatus 32 (S78). The force sensation control unit 366 presents the force sensation that is based on the target position or target movement amount on the force sensation presentation apparatus 36 (S80).

According to the fourth embodiment, for example, the same operational support as that in the comparative example shown in Fig. 9(b) can be provided. According to the comparative example shown in Fig. 9(b), an operation trajectory C2 made by the operator can be more stabilized compared to the comparative example shown in Fig. 9(a).

In the above embodiments, an explanation is given regarding a case where support is provided using both visual and force sensations. In another embodiment, only one of visual and force sensations may be provided, or support may be provided using only a part of the above-described operation support information.

In the above embodiments, an explanation is given regarding a case where the operation of a manipulator performed by the user is supported based on the target position or target movement amount of the manipulator. In another embodiment, the position of the manipulator may be automatically controlled based on the target position or target movement amount of the manipulator. For example, the drive control unit 54 may automatically move the manipulator 16 based on the target position or target movement amount of the manipulator 16 regardless of a user operation.

In the above-described embodiments, an explanation is given regarding a case of supporting rotation operations around the x-axis as shown in Fig. 3(b). In another embodiment, rotation operations around the z-axis as shown in Fig. 3(c) may be supported. For example, by using a trained model that uses rotation operations around the z-axis performed by a skilled operator as teaching data, operation support information for supporting rotation operations around the z-axis can be generated. In yet another embodiment, operation support information for supporting any other operation may be generated, and support through visual or force sensations may be provided based on the generated operation support information. When supporting multiple types of operations, the type of operation to be supported may be manually switched in response to an input operation by the user.

Described above is an explanation based on the embodiments of the present disclosure. It will be understood by those skilled in the art that the present disclosure is not limited to the above-described embodiments, that various design changes are possible, that various modifications are possible, and that such modifications are also within the scope of the present disclosure.

The present disclosure may be expressed by the following numbered aspects:
1. A manipulation system including:
   a manipulator that manipulates a sample;
   a manipulator drive mechanism that moves the manipulator;
   an imaging apparatus that images the sample and the manipulator through an objective lens; and
   a control apparatus,
   wherein the control apparatus includes:
      a movement amount acquisition unit that acquires a movement amount of the manipulator;
      a position identification unit that identifies a position of the sample and a position of the manipulator based on an image captured by the imaging apparatus;
      a first calculation unit that uses the position of the sample and the movement amount of the manipulator as input and calculates a first movement amount of the manipulator for performing a predetermined operation on the sample;
      a second calculation unit that uses the position of the manipulator as input and calculates a second movement amount of the manipulator for guiding the manipulator to a reference trajectory set around the sample; and
      a third calculation unit that uses the first and second movement amounts so as to calculate a target movement amount of the manipulator.
2. The manipulation system according to the first aspect,
   wherein the first calculation unit uses, as input, time-series data in which the position of the sample and the position of the manipulator are associated with each other in time series and calculates the first movement amount.
3. The manipulation system according to the first or second aspect,
   wherein the first calculation unit calculates the first movement amount by using a trained model that has undergone machine-learning using teaching data in which the position of the sample and the movement amount of the manipulator are associated with each other in time series, which are acquired when a skilled operator performs the predetermined operation.
4. The manipulation system according to any one of the first through third aspects,
   wherein the reference trajectory is set based on a probability density function that represents distribution of positions of the manipulator acquired when a skilled operator performs the predetermined operation.
5. The manipulation system according to any one of the first through fourth aspects,
   wherein the third calculation unit calculates the target movement amount by weight-averaging the first movement amount and the second movement amount by using a weight coefficient according to a distance from the reference trajectory to the position of the manipulator.
6. The manipulation system according to any one of the first through fifth aspects, further including:
   a display apparatus that displays operation support information that is based on the target movement amount.
7. The manipulation system according to the third aspect,
   wherein the first calculation unit further calculates the reliability level of the first movement amount by using the trained model, the manipulation system further including:
   a display apparatus that displays operation support information that is based on the target movement amount and the reliability level.
8. The manipulation system according to the sixth or seventh aspect,
   wherein the display apparatus further displays the reference trajectory.
9. The manipulation system according to any one of the first through eighth aspects, further including:
   a force sensation presentation apparatus that is configured to present a force sensation according to the target movement amount to a user.
10. The manipulation system according to the ninth aspect,
   wherein the force sensation presentation apparatus performs:
   presenting a force sensation with a magnitude of force of a constant value regardless of the magnitude of the target movement amount if the distance from the reference trajectory to the current position of the manipulator is equal to or greater than a predetermined value; and
   presenting a force sensation with a magnitude of force that is variable according to the magnitude of the target movement amount if the distance from the reference trajectory to the current position of the manipulator is less than the predetermined value.
11. The manipulation system according to the seventh aspect, further including:
   a force sensation presentation apparatus that is configured to present a force sensation a user,
   wherein the force sensation presentation apparatus performs:
      presenting a force sensation with a magnitude of force that is variable according to the magnitude of the target movement amount if the target movement amount is smaller than the reliability level; and
      presenting a force sensation with a magnitude of force of a constant value regardless of the magnitude of the target movement amount if the target movement amount is larger than the reliability level.
12. The manipulation system according to any one of the first through eleventh aspects,
   wherein the manipulator drive mechanism moves the manipulator based on the target movement amount.
13. A manipulation method including:
   acquiring an image of a sample and a manipulator captured through an objective lens;
   acquiring a movement amount of the manipulator;
   identifying a position of the sample and a position of the manipulator based on the image acquired;
   using the position of the sample and the movement amount of the manipulator as input and calculating a first movement amount of the manipulator for performing a predetermined operation on the sample;
   using the position of the manipulator as input and calculating a second movement amount of the manipulator for guidance to a reference trajectory set around the sample; and
   using the first and second movement amounts so as to calculate a target movement amount of the manipulator.
14. A program or a non-transitory recording medium having embodied thereon the program comprising computer-implemented modules including:
   a module that acquires an image of a sample and a manipulator captured through an objective lens;
   a module that acquires a movement amount of the manipula-to r;
   a module that identifies a position of the sample and a position of the manipulator based on the image acquired;
   a module that uses the position of the sample and the movement amount of the manipulator as input and calculates a first movement amount of the manipulator for performing a predetermined operation on the sample;
   a module that uses the position of the manipulator as input and calculates a second movement amount of the manipulator for guidance to a reference trajectory set around the sample; and
   a module that uses the first and second movement amounts so as to calculate a target movement amount of the manipulator.
15. A manipulation system including:
   a manipulator that manipulates a sample;
   a manipulator drive mechanism that moves the manipulator;
   an imaging apparatus that images the sample and the manipulator through an objective lens; and
   a control apparatus,
   wherein the control apparatus includes:
      a position identification unit that identifies a position of the sample and a position of the manipulator based on an image captured by the imaging apparatus;
      a calculation unit that uses the position of the sample and the position of the manipulator as input and calculates a target position of the manipulator for performing a predetermined operation on the sample and the reliability level of the target position; and
      a display apparatus that displays operation support information that is based on the target position and the reliability level.
16. The manipulation system according to the fifteenth aspect, further including:
   a force sensation presentation apparatus that is configured to present a force sensation a user,
   wherein the force sensation presentation apparatus performs:
      presenting a force sensation with a magnitude of force of a constant value regardless of the distance from the current position to the target position if the current position is outside a confidence circle having the target position as a center thereof and the reliability level as a radius thereof, and
      presenting a force sensation with a magnitude of force that is variable according to the distance from the current position to the target position if the current position is inside the confidence circle.
17. A manipulation method including:
   acquiring an image of a sample and a manipulator captured through an objective lens;
   identifying a position of the sample and a position of the manipulator based on the image acquired;
   using the position of the sample and the position of the manipulator as input and calculating a target position of the manipulator for performing a predetermined operation on the sample and the reliability level of the target position; and
   displaying operation support information that is based on the target position and the reliability level.
18. A program or a non-transitory recording medium having embodied thereon the program comprising computer-implemented modules including:
   a module that acquires an image of a sample and a manipulator captured through an objective lens;
   a module that identifies a position of the sample and a position of the manipulator based on the image acquired;
   a module that uses the position of the sample and the position of the manipulator as input and calculates a target position of the manipulator for performing a predetermined operation on the sample and the reliability level of the target position; and
   a module that displays operation support information that is based on the target position and the reliability level.

### INDUSTRIAL APPLICABILITY

According to the present disclosure, the operation accuracy when performing a predetermined operation on a sample using a manipulator can be improved.

### REFERENCE SIGNS LIST

10, 110, 210, 310 ... manipulation system, 16 ... manipulator, 20 ... objective lens, 24 ... imaging apparatus, 26 ... manipulator drive mechanism, 30, 130, 230, 330 ... control apparatus, 32 ... display apparatus, 36 ... force sensation presentation apparatus, 40 ... sample, 56 ... movement amount acquisition unit, 58 ... position identification unit, 70, 270 ... first calculation unit, 72 ... second calculation unit, 74, 274 ... third calculation unit, 176, 376 ... calculation unit, 80 ... reference trajectory

## Claims

1. A manipulation system comprising:
a manipulator for manipulating a sample;
a manipulator drive mechanism for moving the manipulator;
an imaging apparatus that images the sample and the manipulator through an objective lens; and
a control apparatus,
wherein the control apparatus includes:
a movement amount acquisition unit that acquires a movement amount of the manipulator;
a position identification unit that identifies a position of the sample and a position of the manipulator based on an image captured by the imaging apparatus; and
a calculation unit that calculates a target position or target movement amount of the manipulator for performing a predetermined operation on the sample based on the position of the sample and the position or movement amount of the manipulator.

2. The manipulation system according to Claim 1,
wherein the calculation unit calculates the target position or the target movement amount based on time-series data in which the position of the sample and the position or movement amount of the manipulator are associated with each other in time series.

3. The manipulation system according to Claim 1,
wherein the calculation unit calculates the target position or the target movement amount by using a trained model that has undergone machine-learning using teaching data in which the position of the sample and the position or movement amount of the manipulator are associated with each other in time series, which are acquired when a skilled operator performs the predetermined operation.

4. The manipulation system according to any one of Claims 1 through 3,
wherein the calculation unit calculates the target movement amount based on the position of the sample and the movement amount of the manipulator.

5. The manipulation system according to Claim 4,
wherein the calculation unit includes:
a first calculation unit that uses the position of the sample and the movement amount of the manipulator as input and calculates a first movement amount for performing a predetermined operation on the sample;
a second calculation unit that uses the position of the manipulator as input and calculates a second movement amount of the manipulator for guiding the manipulator to a reference trajectory set around the sample; and
a third calculation unit that uses the first and second movement amounts so as to calculate the target movement amount of the manipulator.

6. The manipulation system according to Claim 5,
wherein the reference trajectory is set based on a probability density function that represents distribution of positions of the manipulator acquired when a skilled operator performs the predetermined operation.

7. The manipulation system according to Claim 5 or 6,
wherein the third calculation unit calculates the target movement amount by weight-averaging the first movement amount and the second movement amount by using a weight coefficient according to a distance from the reference trajectory to the position of the manipulator.

8. The manipulation system according to any one of Claims 5 through 7, further comprising:
a display apparatus that displays operation support information that is based on the target movement amount and the reference trajectory.

9. The manipulation system according to any one of Claims 5 through 7,
wherein the calculation unit further calculates the reliability level of the first movement amount, the manipulation system further comprising:
a display apparatus that displays operation support information that is based on the target movement amount and the reliability level.

10. The manipulation system according to any one of Claims 1 through 3, further comprising:
a display apparatus that displays operation support information that is based on the target position and the target movement amount.

11. The manipulation system according to any one of Claims 1 through 3,
wherein the calculation unit further calculates the reliability level of the target position or the target movement amount, the manipulation system further comprising:
a display apparatus that displays operation support information that is based on the target position or the target movement amount and on the reliability level.

12. The manipulation system according to any one of Claims 1 through 11, further comprising:
a force sensation presentation apparatus that is configured to present a force sensation according to the target position or the target movement amount to a user.

13. The manipulation system according to any one of Claims 5 through 9, further comprising:
a force sensation presentation apparatus that is configured to present a force sensation according to the target movement amount to a user,
wherein force sensation presentation apparatus performs:
presenting a force sensation with a magnitude of force of a constant value regardless of the magnitude of the target movement amount if the distance from the reference trajectory to the current position of the manipulator is equal to or greater than a predetermined value; and
presenting a force sensation with a magnitude of force that is variable according to the magnitude of the target movement amount if the distance from the reference trajectory to the current position of the manipulator is less than the predetermined value.

14. The manipulation system according to Claim 9, further comprising:
a force sensation presentation apparatus that is configured to present a force sensation a user,
wherein the force sensation presentation apparatus performs:
presenting a force sensation with a magnitude of force that is variable according to the magnitude of the target movement amount if the target movement amount is smaller than the reliability level; and
presenting a force sensation with a magnitude of force of a constant value regardless of the magnitude of the target movement amount if the target movement amount is larger than the reliability level.

15. The manipulation system according to Claim 11, further comprising:
a force sensation presentation apparatus that is configured to present a force sensation a user,
wherein the force sensation presentation apparatus performs:
presenting a force sensation with a magnitude of force of a constant value regardless of the distance from the current position to the target position if the current position is outside a confidence circle having the target position as a center thereof and the reliability level as a radius thereof, and
presenting a force sensation with a magnitude of force that is variable according to the distance from the current position to the target position if the current position is inside the confidence circle.

16. The manipulation system according to any one of Claims 1 through 15,
wherein the manipulator drive mechanism moves the manipulator based on the target position or the target movement amount.

17. A manipulation method comprising:
acquiring an image of a sample and a manipulator captured through an objective lens;
acquiring a movement amount of the manipulator;
identifying a position of the sample and a position of the manipulator based on the image acquired;
calculating a target position or target movement amount of the manipulator for performing a predetermined operation on the sample based on the position of the sample and the position or movement amount of the manipulator.

18. A program comprising computer-implemented modules including:
a module that acquires an image of a sample and a manipulator captured through an objective lens;
a module that acquires a movement amount of the manipulator;
a module that identifies a position of the sample and a position of the manipulator based on the image acquired; and
a module that calculates a target position or target movement amount of the manipulator for performing a predetermined operation on the sample based on the position of the sample and the position or movement amount of the manipulator.
